# EUROPÄISCHE PATENTSCHRIFT

(11) **EP 0 859 767 B1**
(45) Veröffentlichungstag und Bekanntmachung des Hinweises auf die Patenterteilung: **11.10.2000**
(21) Anmeldenummer: 96937251.5
(22) Anmeldetag: 24.10.1996
(51) Int. Cl.: C07D 271/06, C07D 271/10, C07D 413/04, A01N 43/824, A01N 43/836

(54) **FLUORPROPENYLOXADIAZOLE UND IHRE VERWENDUNG ALS SCHÄDLINGSBEKÄMPFUNGSMITTEL**
FLUOROPROPENYL OXADIAZOLES AND THE USE THEREOF AS PEST CONTROL AGENTS
FLUOROPROPENYLOXADIAZOLES ET LEUR UTILISATION COMME PESTICIDES

(30) Priorität: 06.11.1995 DE 19541261
(43) Veröffentlichungstag der Anmeldung: 26.08.1998
(73) Patentinhaber: BAYER AG, 51368 Leverkusen (DE)
(72) Erfinder: KRÄMER, Wolfgang, D-51399 Burscheid (DE); KRAATZ, Udo, D-51375 Leverkusen (DE); ANDERSCH, Wolfram, D-51469 Bergisch Gladbach (DE); ERDELEN, Christoph, D-42799 Leichlingen (DE); TURBERG, Andreas, D-40699 Erkrath (DE); MENCKE, Norbert, D-51381 Leverkusen (DE)
(86) Internationale Anmeldenummer: EP9604663
(87) Internationale Veröffentlichungsnummer: WO9717335

(56) Entgegenhaltungen:
- EP-A- 0 290 379
- US-A- 4 952 580
- JOURNAL OF AGRICULTURAL AND FOOD CHEMISTRY, Bd. 31, Nr. 2, 1983, WASHINGTON DC, US, Seiten 250-3, XP002024234 T. ANDO ET AL.: "3-(3,3-Dihal-2-propenyl) analogues of allethrin and related pyrethroids: synthesis, biological activity, and photostability"

## Beschreibung

Die vorliegende Erfindung betrifft neue Fluorpropenylheterocyclen, Verfahren zu ihrer Herstellung und ihre Verwendung zur Bekämpfung von tierischen Schädlingen, insbesondere von Insekten, Spinnentieren und Nematoden, die in der Landwirtschaft, in den Forsten, im Vorrats- und Materialschutz sowie auf dem Hygienesektor vorkommen.

Es ist bereits bekannt geworden, daß bestimmte Fluorbutenylverbindungen als Nematizide wirksam sind (vgl. z.B. WO 88/00183). Ebenso ist bekannt, daß 2-Mercapto-5-pyrazinyl-1,3,4-oxadiazole und -1,3,4-thiadiazole eine nematizide Wirkung aufweisen (EP 0 290 379). Die Wirksamkeit und Wirkungsbreite dieser Verbindungen ist jedoch insbesondere bei niedrigen Aufwandmengen und Konzentrationen nicht immer völlig zufriedenstellend.

Es wurden nun neue Verbindungen der Formel (I) gefunden

CF₂=CX-CH₂-Het (I),

in welcher
- X: für Wasserstoff oder Halogen steht,
- Het: für einen der Reste 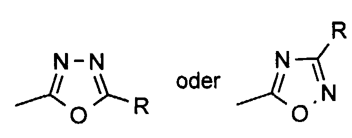 steht und
- R: für jeweils gegebenenfalls substituiertes Alkyl, Aryl, Aralkyl oder Hetaryl steht.

Weiterhin wurde gefunden, daß man die Verbindungen der Formel (I) erhält, wenn man
A) Amidoxime der Formel (II) 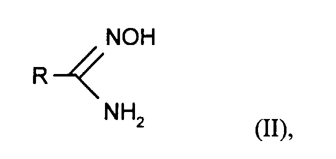 in welcher
   - R: die oben angegebene Bedeutung hat,
   mit Säurechloriden der Formel (III)

   CF₂=CX-CH₂-COCl (III),

   in welcher
   - X: die oben angegebene Bedeutung hat,
   gegebenenfalls in Gegenwart eines Verdünnungsmittels und gegebenenfalls in Gegenwart einer Base umsetzt und die so erhaltenen Zwischenprodukte der Formel (IV) 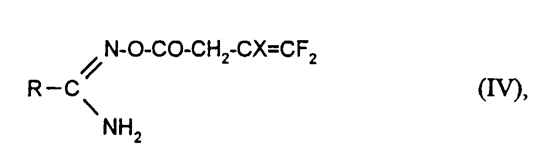 in welcher
   R und X die oben angegebene Bedeutung haben,
   gegebenenfalls isoliert und gegebenenfalls in Gegenwart einer Base cyclisiert,
   oder
B) Carbonsäurehydrazide der Formel (V)

   R-CONH-NH₂ (V),

   in welcher
   - R: die oben angegebene Bedeutung hat,
   mit Säurechloriden der Formel (III)

   CF₂=CX-CH₂-COCl (III),

   in welcher
   - X: für Wasserstoff oder Halogen steht,
   in Gegenwart eines Verdünnungsmittels und in Gegenwart einer Base zu Verbindungen der Formel (VI)

   R-CONH-NH-CO-CH₂-CX=CF₂ (VI)

   in welcher
   R und X die oben angegebene Bedeutung haben,
   umsetzt, diese gegebenenfalls isoliert und gegebenenfalls in Gegenwart eines Verdünnungsmittels und gegebenenfalls in Gegenwart eines wasserabspaltenden Mittels cyclisiert.

Schließlich wurde gefunden, daß die neuen Verbindungen der Formel (I) stark ausgeprägte biologische Eigenschaften besitzen und vor allem zur Bekämpfung von tierischen Schädlingen, insbesondere von Insekten, Spinnentieren und Nematoden, die in der Landwirtschaft, in Forsten, im Vorrats- und Materialschutz sowie auf dem Hygienesektor vorkommen, geeignet sind.

Die erfindungsgemäßen Verbindungen sind durch die Formel (I) allgemein definiert.

Bevorzugte Substituenten bzw. Bereiche der in den oben und nachstehend erwähnten Formeln aufgeführten Reste werden im folgenden erläutert.
- X: steht bevorzugt für Wasserstoff, Fluor oder Chlor.
- Het: steht bevorzugt für einen der Reste 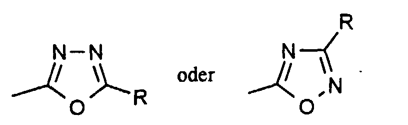
- R: steht bevorzugt für C₁-C₈-Alkyl, C₁-C₈-Halogenalkyl, C₁-C₈-Alkoxy-C₁-C₈-alkyl, C₁-C₈-Alkylthio-C₁-C₈-alkyl, Phenoxy-C₁-C₄-alkyl, welches gegebenenfalls im Phenylteil substituiert ist durch Halogen, C₁-C₆-Alkyl, C₁-C₆-Alkoxy, C₁-C₆-Halogenalkyl oder C₁-C₆-Halogenalkoxy,
für gegebenenfalls durch Halogen, C₁-C₆-Alkyl, C₁-C₆-Alkoxy, C₁-C₆-Alkylthio, C₁-C₆-Halogenalkyl, C₁-C₈-Alkoxycarbonyl, Aminocarbonyl, C₁-C₆-Alkylaminocarbonyl, Di-C₁-C₆-Alkylaminocarbonyl, Nitro, Cyano oder SCN substituiertes Phenyl,
für gegebenenfalls im Phenylteil durch Halogen, C₁-C₄-Alkyl, C₁-C₄-Halogenalkyl, C₁-C₄-Alkoxy oder C₁-C₄-Alkylthio substituiertes Phenyl-C₁-C₄-alkyl oder
für gegebenenfalls benzokondensiertes und gegebenenfalls durch Halogen, C₁-C₆-Alkyl, C₁-C₆-Halogenalkyl oder Phenyl (welches gegebenenfalls durch Halogen, C₁-C₆-Alkyl, C₁-C₄-Alkoxy, C₁-C₄-Halogenalkyl oder C₁-C₄-Halogenalkoxy substituiert ist) substituiertes 5- oder 6-gliedriges Hetaryl mit ein oder zwei Heteroatomen aus der Reihe Sauerstoff, Schwefel und Stickstoff.
- X: steht besonders bevorzugt für Wasserstoff oder Fluor.
- Het: steht besonders bevorzugt für einen der Reste 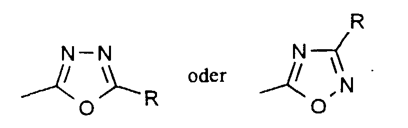
- R: steht besonders bevorzugt für C₁-C₄-Alkyl, C₁-C₃-Halogenalkyl, C₁-C₄-Alkoxy-C₁-C₄-alkyl, C₁-C₄-Alkylthio-C₁-C₄-alkyl, Phenoxy-C₁-C₂-alkyl, welches gegebenenfalls im Phenylteil substituiert ist durch Fluor, Chlor, C₁-C₄-Alkyl, C₁-C₄-Alkoxy, C₁-C₃-Halogenalkyl oder C₁-C₃-Halogenalkoxy,
für gegebenenfalls durch Fluor, Chlor, C₁-C₄-Alkyl, C₁-C₄-Alkoxy, C₁-C₄-Alkylthio, Nitro oder Cyano substituiertes Phenyl,
für gegebenenfalls im Phenylteil durch Fluor, Chlor, C₁-C₄-Alkyl oder C₁-C₂-Halogenalkyl substituiertes Phenyl-C₁-C₂-alkyl oder
für jeweils gegebenenfalls benzokondensiertes und gegebenenfalls durch Fluor, Chlor, C₁-C₄-Alkyl, C₁-C₂-Halogenalkyl oder Phenyl (welches gegebenenfalls durch Halogen, C₁-C₄-Alkyl, C₁-C₂-Halogenalkyl, C₁-C₂-Alkoxy oder C₁-C₂-Halogenalkoxy substituiert ist) substituiertes Furanyl, Thienyl oder Pyridyl.
- X: steht ganz besonders bevorzugt für Fluor.
- Het: steht ganz besonders bevorzugt für einen der Reste 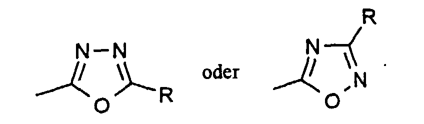
- R: steht ganz besonders bevorzugt für C₁-C₃-Alkyl, Chlormethyl,Trifluormethyl, C₁-C₄-Alkoxymethyl, C₁-C₄-Alkylthiomethyl, Phenoxymethyl, welches gegebenenfalls im Phenylteil substituiert ist durch Fluor, Chlor, C₁-C₃-Alkyl, C₁-C₃-Alkoxy, Trifluormethyl oder Trifluormethoxy,
für gegebenenfalls durch Fluor, Chlor, C₁-C₃-Alkyl, C₁-C₃-Alkoxy oder C₁-C₃-Alkylthio substituiertes Phenyl,
für gegebenenfalls im Phenylteil durch Fluor, Chlor, C₁-C₃-Alkyl oder Trifluormethyl substituiertes Benzyl oder
für jeweils gegebenenfalls durch Fluor, Chlor, C₁-C₃-Alkyl oder Trifluormethyl oder Phenyl (welches gegebenenfalls durch Fluor, Chlor, C₁-C₃-Alkyl, Trifluormethyl oder Trifluormethoxy substituiert ist) substituiertes Furanyl, Thienyl oder Pyridyl.
- X: steht besonders hervorgehoben für Fluor.
- Het: steht besonders hervorgehoben für einen der Reste 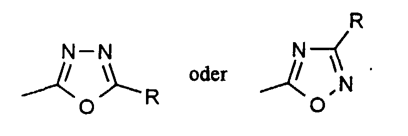
- R: steht besonders hervorgehoben für gegebenenfalls durch Fluor, Chlor, Methyl, Ethyl, Methoxy oder Ethoxy substituiertes Phenyl oder
für jeweils gegebenenfalls durch Fluor, Chlor, Methyl oder Ethyl substituiertes Furanyl, Thienyl oder Pyridyl.

Die oben aufgeführten allgemeinen oder in Vorzugsbereichen aufgeführten Restedefinitionen bzw. Erläuterungen gelten für die Endprodukte und für die Ausgangs- und Zwischenprodukte entsprechend. Diese Restedefinitionen können untereinander, also auch zwischen den jeweiligen Vorzugsbereichen, beliebig kombiniert werden.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Erfindungsgemäß besonders hevorgehoben sind die Verbindungen der Formel (I), in welchem eine Kombination der vorstehend als besonders hevorgehoben aufgeführten Bedeutungen vorliegt.

In den oben und nachstehend aufgeführten Restedefinitionen sind Kohlenwasserstoffreste, wie Alkyl oder Alkenyl - auch in Verbindung mit Heteroatomen wie Alkoxy oder Alkylthio - soweit möglich jeweils geradkettig oder verzweigt.

Verwendet man bei der Herstellung von Verbindungen der Formel (I) gemäß Verfahren A) 4-Methylbenzoesäure-amidoxim und 3,4,4-Trifluorbut-3-en-säurechlorid als Ausgangsstoffe, so kann der Reaktionsverlauf durch folgendes Reaktionsschema wiedergegebenen werden:

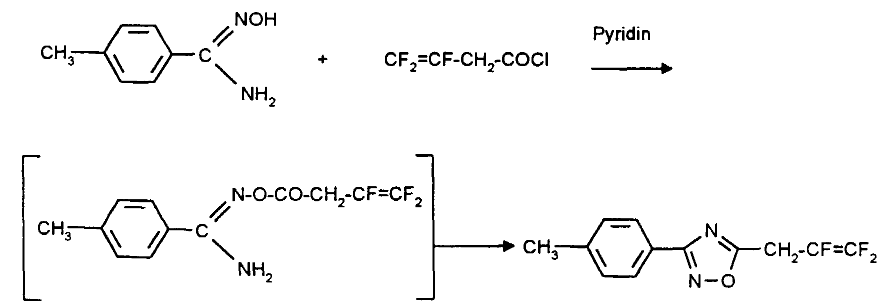

Verwendet man der Herstellung von Verbindungen der Formel (I) gemäß Verfahren B) 4-Chlorbenzhydrazid und 3,4,4-Trifluorbut-3-en-säurechlorid als Ausgangsstoffe, so kann der Reaktionsverlauf durch folgendes Reaktionsschema wiedergegeben werden:

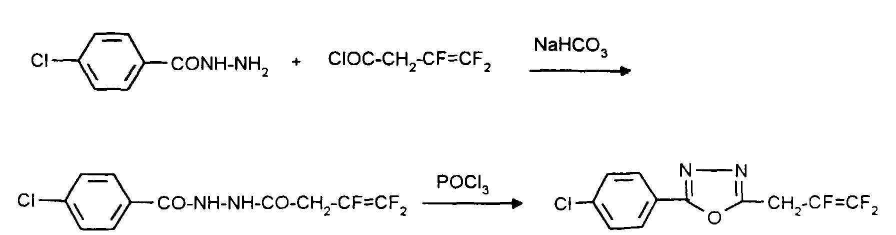

Das erfindungsgemäße Verfahren A) zur Herstellung von Verbindungen der Formel (I) ist dadurch gekennzeichnet, daß man zunächst Amidoxime der Formel (II) mit Säurechloriden der Formel (III) gegebenenfalls in Gegenwart einer Base umsetzt.

Als Verdünnungsmittel kommen hierfür organische Lösungsmittel in Frage. Genannt seien Kohlenwasserstoffe wie Cyclohexan, Toluol oder Benzol, halogenierte, insbesondere chlorierte Kohlenwasserstoffe wie Methylenchlorid, Chloroform, Dichlorethan oder Chlorbenzol und ferner Nitrile wie Acetonitril.

Die Umsetzung der Amidoxime der Formel (II) mit den Säurechloriden der Formel (III) wird vorzugsweise in Gegenwart einer Base durchgeführt. Als Base kommen sowohl organische Basen, insbesondere tertiäre Amine wie Diazabicycloundecan (DBU), Diazabicyclononen (DBN), Diazabicyclooctan (DABCO), Pyridin oder Triethylenamin, als auch anorganische Basen, insbesondere Alkalimetall- oder Erdalkalimetallcarbonate, -hydrogencarbonate, -hydroxide oder -oxide wie Kaliumcarbonat, Natriumhydrogencarbonat, Natriumhydroxid oder Calciumoxid in Frage, Es ist im allgemeinen nicht notwendig, die Zwischenprodukte der Formel (IV) zu isolieren. Diese lassen sich zum Beispiel durch längeres Erwärmen und/oder in Gegenwart der genannten Basen in die gewünschten 1,2,4-Oxadiazole überführen.

Die Reaktionstemperatur kann beim erfindungsgemäßen Verfahren A) in einem größeren Bereich variiert werden. Im allgemeinen arbeitet man bei Temperaturen zwischen -10°C und 180°C, bevorzugt zwischen 0°C und 140°C.

Im allgemeinen setzt man die Ausgangsstoffe der Formeln (II) und (III) in etwa äquivalenten Mengen ein, es ist jedoch auch möglich, die eine oder andere Komponente in einem größeren Überschuß (bis etwa 2:1) einzusetzen.

Man setzt in der Regel auch eine mindestens äquivalente Menge der Base zu.

Das erfindungsgemäße Verfahren A) wird im allgemeinen unter Normaldruck durchgeführt (vgl. auch Houben-Weyl, Methoden der organischen Chemie, Bd E 8c, Teil 3, S. 409 ff)

Das erfindungsgemäße Verfahren B) zur Herstellung von Verbindungen der Formel (I) ist dadurch gekennzeichnet, daß man Carbonsäurehydrazide der Formel (II) zunächst mit Säurechloriden der Formel (III) in Gegenwart eines Verdünnungsmittels und in Gegenwart einer Base zu den Diacylhydrazinen der Formel (VI) umsetzt (Schritt 1), diese gegebenenfalls isoliert und gegebenenfalls in Gegenwart eines Verdünnungsmittels und gegebenenfalls in Gegenwart eines wasserabspaltenden Mittels cyclisiert (Schritt 2).

Als Verdünnungsmittel für den Schritt 1 des erfindungsgemäßen Verfahren B) kommen organische Lösungsmittel in Frage. Genannt seien aliphatische oder aromatische, gegebenenfalls halogenierte (insbesondere chlorierte) Kohlenwasserstoffe wie Cyclohexan, Toluol, Xylol, Dichlormethan, Trichlormethan, Dichlorethan oder Chlorbenzol, Ether wie Tetrahydrofuran oder Dioxan, Nitrile wie Acetonitril, Amide wie Dimethylformamid oder Sulfoxide wie Dimethylsulfoxid.

Die Umsetzung kann auch in einem Zweiphasensystem bestehend aus Wasser und einem organischen Lösungsmittel wie Wasser/Dichlormethan oder Wasser/Toluol durchgeführt werden.

Als Base für den Schritt 1 des erfindungsgemäßen Verfahren B) kommen sowohl organische Basen, insbesondere tertiäre Amine wie DBU, DBN, DABCO, Pyridin oder Triethylamin, als auch anorganische Basen, insbesondere Alkalimetallcarbonate, -hydrogencarbonate oder -hydroxide wie Natriumcarbonat, Kaliumcarbonat, Natriumhydrogencarbonat oder Natriumhydroxid in Frage.

Die Zwischenprodukte der Formel (VI) können vor der Durchführung, von Schritt 2 (Cyclisierung) des erfindungsgemäßen Verfahrens B) isoliert werden. Es ist aber auch möglich, diese Schritte direkt im Anschluß an Schritt 1, d.h. ohne Isolierung der Zwischenprodukte der Formel (VI) durchzuführen.

Schritt 2 des erfindungsgemäßen Verfahrens B) (vgl. Houben-Weyl, Methoden der org. Chemie, Bd. E 8c, Teil 3, S. 563 ff) wird vorzugsweise in Gegenwart eines Verdünnungsmittels durchgeführt. Als Verdünnungsmittel kommen inerte organische Lösungsmittel, beispielsweise gegebenenfalls halogenierte (chlorierte) Kohlenwasserstoffe wie Toluol, Xylol oder Dichlorbenzol oder Amide wie Dimethylacetamid in Frage.

Schritt 2 des erfindungsgemäßen Verfahrens B) wird in Gegenwart eines wasserabspaltenden Mittels durchgeführt. Als solche kommen die üblichen wasserabspaltenden Mittel in Frage. Genannt seien beispielsweise Phosphoroxidtrichlorid, Polyphosphorsäure, p-Toluolsulfonsäure und Phosphorpentoxid.

Es kann von Vorteil sein, die Umsetzung in einer Apparatur mit Wasserabscheider durchzuführen.

Die Reaktionstemperatur kann beim erfindungsgemäßen Verfahren B) innerhalb eines größeren Bereichs variiert werden. Im allgemeinen arbeitet man bei Temperaturen zwischen 0°C und 200°C, bevorzugt zwischen 20°C und 150°C.

Dabei kann die Temperatur, bei welcher Schritt 1 durchgeführt wird, sich unterscheiden von der Temperatur, bei welcher Schritt 2 durchgeführt wird (vgl. Herstellungsbeispiele).

Im allgemeinen setzt man die Ausgangsstoffe der Formel (III) und (V) in etwa äquivalenten Mengen ein, es ist jedoch auch möglich, die eine oder andere Komponente in einem größeren Überschuß (bis 3:1, bevorzugt bis 1,5:1) einzusetzen.

Man setzt in der Regel auch eine mindestens äquivalente Menge der Base zu.

Das erfindungsgemäße Verfahren B) wird im allgemeinen unter Normaldruck durchgeführt.

Die Zwischenprodukte der Formel (VI) lassen sich auch herstellen, wenn man Carbonsäurehydrazide der Formel (VII)

CF₂=CX-CH₂-CO-NH-NH₂ (VII)

in welcher
- X: die oben angegebene Bedeutung hat,
mit Carbonsäurechloriden der Formel (VIII)

R-COCl (VIII)

in welcher
- R: die oben angegebene Bedeutung hat,
umsetzt.

Diese Umsetzung kann unter den oben für Schritt 1 des erfindungsgemäßen Verfahrens B) beschriebenen Reaktionsbedingungen durchgeführt werden.

Die als Ausgangsstoffe benötigten Amidoxime der Formel (II) und die Carbonsäurehydrazide der Formel (V) sind bekannt und/oder können nach allgemein bekannten Verfahren hergestellt werden (s. z.B. Houben-Weyl, Methoden der organischen Chemie, Bd. (VIII), S. 676; F. Eloy, R. Lenaers, Chem. Rev. 62, 155 (1962)).

Die als Ausgangsstoffe weiterhin benötigten Carbonsäurechloride der Formel (III) sind bekannt (s. z.B. US 5 389 680 und EP 0 432 861).

Die Wirkstoffe eignen sich zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Spodoptera litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp..

Die erfindungsgemäßen Verbindungen der Formel (I) zeichnen sich insbesondere durch eine hervorragende nematizide Wirkung, beispielsweise gegen Meloidogyne incognita aus. Sie zeigen eine gute blattinsektizide Wirkung.

Die erfindungsgemäßen Wirkstoffe besitzen systemische Wirkung.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-DichloroN-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino-[alpha-(o-tolyloxy)-o-tolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox, Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon, Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin, Lambda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram,
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen, Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, -Triazophos, Triazuron, Trichlorfon, Triflumuron, Trimethacarb,
Vamidothion, XMC, Xylylcarb, YI 5301 / 5302, Zetamethrin.

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp.

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola SPP.

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.

Aus der Ordnung der Siphonapterida z.B. Pulex spp., Ctenocephalides spp., Xenopsylla spp., Ceratophyllus spp.

Aus der Ordnung der Heteropterida z.B. Cimex spp., Triatoma spp., Rhodnius spp., Panstrongylus spp.

Aus der Ordnung der Blattarida z.B. Blatta orientalis, Periplaneta americana, Blattela germanica, Supella spp.

Aus der Unterklasse der Acaria (Acarida) und den Ordnungen der Meta- sowie Mesostigmata z.B. Argas spp., Ornithodorus spp., Otabius spp., Ixodes spp., Amblyomma spp., Boophilus spp., Dermacentor spp., Haemaphysalis spp., Hyalomma spp., Rhipicephalus spp., Dermanyssus spp., Raillietia spp., Pneumonyssus spp., Sternostoma spp., Varroa spp.

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Die Herstellung und die Verwendung der erfindungsgemäßen Wirkstoffe gehen aus den nachfolgenden Beispielen hervor.

### Herstellungsbeispiele

### Beispiel 1

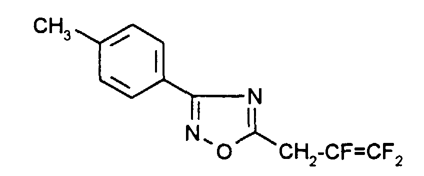

Zur Lösung von 6,0 g (40 mmol) 4-Methylbenzoesäureamidoxim in 60 ml Dioxan und 4,9 g (62 mmol) Pyridin tropft man bei 20°C 6,0 g (38 mmol) 3,4,4-Trifluor-but-en-säurechlorid zu und rührt anschließend 12 h bei 90°C. Man gießt dann auf Wasser, extrahiert das Produkt mit Methylenchlorid und engt die organische Phase im Vakuum ein.

Das erhaltene Rohprodukt wird an Kieselgel im System Chloroform/Essigester = 4:1 chromatographiert. Man erhält 5,5 g (57 % Ausbeute d.Th.) kristallines 3-(4-Methylphenyl)-5-(2,3,3-trifluorprop-2-en-yl)-1,2,4-oxadiazol vom Fp. 46-50°C.

### Beispiel 2

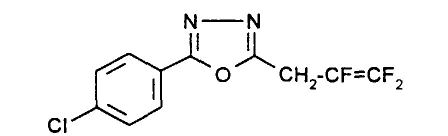

4,0 g (13,7 mmol) 1-(4-Chlorbenzoyl)-2-(3,3,4-trifluor-but-3-enoyl)-hydrazin werden in 50 ml Toluol mit 4,2 g (27,4 mmol) Phosphoroxidtrichlorid 1 h unter Rückfluß gekocht. Anschließend wird das Toluol im Vakuum entfernt und der Rückstand in Methylenchlorid/Eiswasser aufgenommen. Die organische Phase wird im Vakuum eingeengt und mittels Säulenchromatographie an Kieselgel im System Chloroform/Essigester (9:1) gereingt. Man erhält 2,3 g (64,5 % Ausbeute der Theorie) an 2-(4-Chlorphenyl)-5-(2,3,3-trifluorprop-2-enyl)-1,3-4-oxadiazol vom Fp. 72°C.

### Herstellung des Ausgangsprodukts

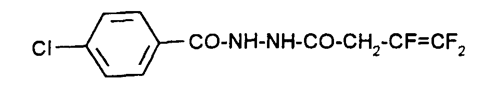

Zu 5,2 g (30,3 mmol) 4-Chlorbenzhydrazid in 50 ml Dichlormethan fügt man eine Lösung von 2,8 g (33 mmol) Natriumhydrogencarbonat in 25 ml Wasser und tropft anschließend unter Rühren bei 0°C 4,8 g (30,3 mmol) 3,4,4-Trifluorbut-3-ensäurechlorid zu. Nach Rühren über Nacht wird das ausgefallene 1-(4-Chlorbenzoyl)-2-(3,4,4-trifluorbut-3-enoyl)-hydrazin abgesaugt.

Ausbeute: 8,0 g (90,4 % d.Th.) vom Fp. 198°C.

Analog zu den Beispielen 1 und 2 bzw. gemäß den allgemeinen Angaben zur Herstellung wurden die in der folgenden Tabelle aufgeführten Verbindungen der Formel (I) erhalten.

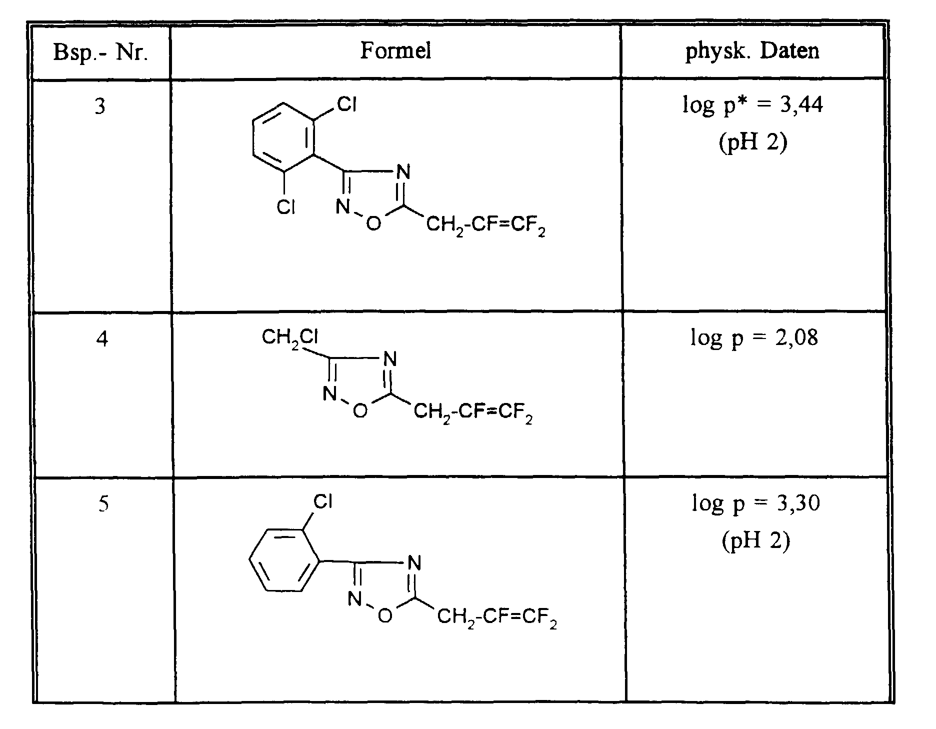

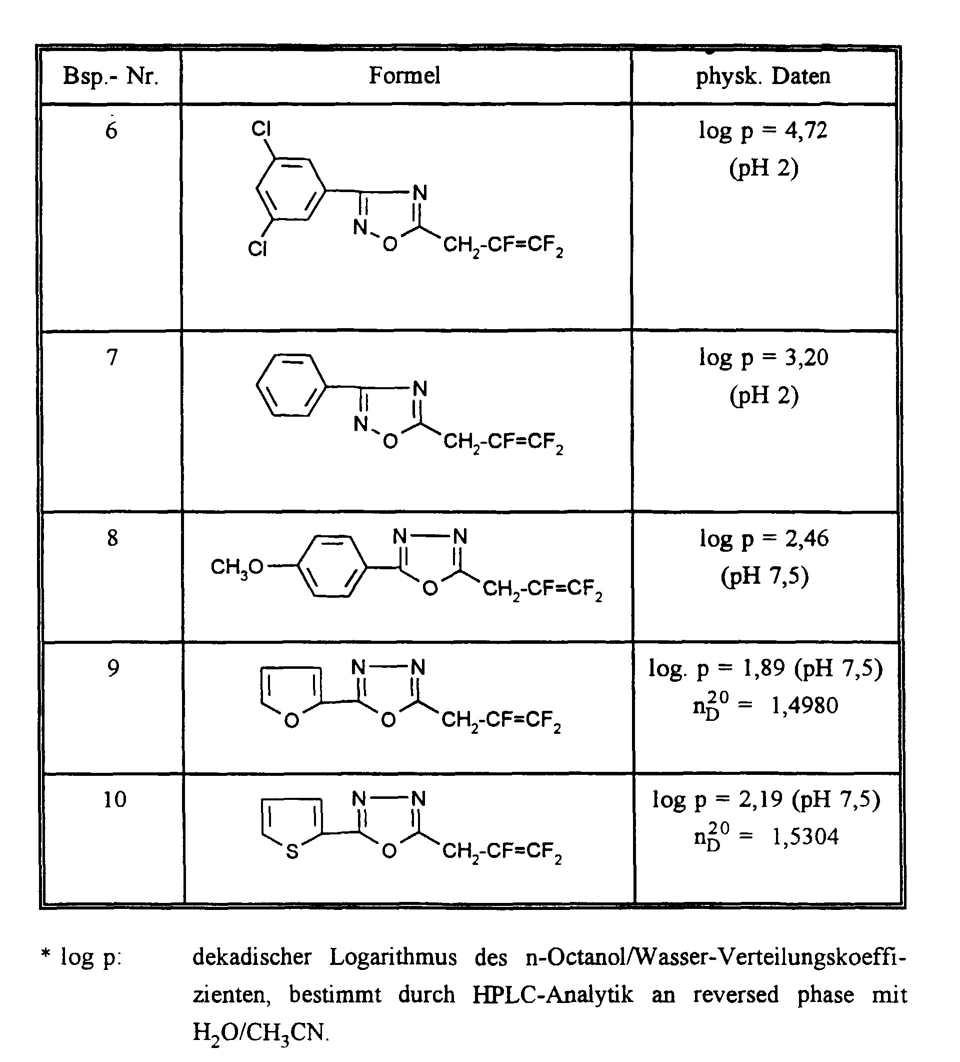

### Anwendungsbeispiel

### Beispiel A

### Grenzkonzentrations-Test / Nematoden

| | |
|---|---|
| Testnematode | Meloidogyne incognita |
| Lösungsmittel | 4 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Test-nematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm (= mg/l) angegeben wird. Man füllt den behandelten Boden in Töpfe, sät Salat ein und hält die Töpfe bei einer Gewächshaus-Temperatur von 25°C.

Nach vier Wochen werden die Salatwurzeln auf Nematodenbefall (Wurzelgallen) untersucht und der Wirkungsgrad des Wirkstoffs in % bestimmt. Der Wirkungsgrad ist 100 %, wenn der Befall vollständig vermieden wird, er ist 0 %, wenn der Befall genau so hoch ist wie bei den Kontrollpflanzen in unbehandeltem, aber in gleicher Weise verseuchtem Boden.

Bei diesem Test besaß z.B. die Verbindung gemäß Herstellungsbeispiel 10 bei einer beispielhaften Wirkstoffkonzentration von 20 ppm einen Wirkungsgrad von 100 %.

### Beispiel B

### Phaedon-Larven-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden, 0 % bedeutet, daß keine Käfer-Larven abgetötet wurden.

In diesem Test bewirken z.B. die Verbindungen gemäß den Herstellungsbeispielen 1, 2 und 7 bei einer beispielhaften Wirkstoffkonzentration von 0,1 % eine Abtötung von 100 % nach 7 Tagen.

### Beispiel C

### Plutella-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella xylostella) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

In diesem Test bewirkten z.B. die Verbindungen gemäß den Herstellungsbeispielen 1, 2, 3, 5, 7 und 8 bei einer bespielhaften Wirkstoffkonzentration von 0,1 % eine Abtötung von mindestens 90 % nach 7 Tagen.

### Beispiel D

### Spodoptera-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

In diesem Test bewirkten z.B. die Verbindungen gemäß den Herstellungsbeispielen 1, 3 und 7 bei einer beispielhaften Wirkstoffkonzentration von 0,1 % eine Abtötung von mindestens 85 % nach 7 Tagen.

### Beispiel E

### Nephotettix-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryzae sativa) werden durch Tauchen in die Wirkstoffzubereitung der gewünschten Konzentration behandelt und mit Larven der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

In diesem Test bewirkten z.B. die Verbindungen gemäß den Herstellungsbeispielen 1, 2 und 3 bei einer beispielhaften Wirkstoffkonzentration von 0,1 % eine Abtötung von 100 % nach 6 Tagen.

### Beispiel F

### Myzus-Test (Wirkungsdauer nach Spritzen)

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnentriebe (Vicia faba), die stark von der schwarzen Bohnenlaus (Aphis fabae) befallen sind, werden durch Tauchen in dieWirkstoffzubereitung der gewünschten Konzentration behandelt.

Nach der gewünschten Zeit wird die Abtötung in % bestimmt. Dabei bedeutet 100 %, daß alle Blattläuse abgetötet wurden, 0 % bedeutet, daß keine Blattläuse abgetötet wurden.

In diesem Test bewirkten z.B. die Verbindungen gemäß den Herstellungsbeispielen 1 bzw. 2 bei einer beispielhaften Wirkstoffkonzentration von 0,1 % eine Abtötung von 90 % bzw. 80 %.

### Beispiel G

### Test mit Fliegenlarven / Entwicklungshemmende Wirkung

| | |
|---|---|
| Testtiere | Alle larvalen Stadien von Lucilia cuprina (OP-restistent) [Puppen und Adulte (ohne Kontakt zum Wirkstoff)] |
| | |
| Lösungsmittel | 35 Gewichtsteile Ethylenglykolmonomethylether |
| | 35 Gewichtsteile Nonylphenolpolyglykolether |

Zwecks Herstellung einer geeigneten Formulierung vermischt man 3 Gewichtsteile Wirkstoff mit 7 Teilen des oben angegebenen Lösungsmittel-Emulgator-Gemisches und verdünnt das so erhaltene Emulsionskonzentrat mit Wasser auf die jeweils gewünschte Konzentration.

30 bis 50 Larven je Konzentration werden auf in Glasröhrchen befindliches Pferdefleisch (1 cm³) gebracht, auf welches 500 µl der zu testenden Verdünnung pipettiert werden. Die Glasröhrchen werden in Kunststoffbecher gestellt, deren Boden mit Seesand bedeckt ist, und im klimatisierten Raum (26°C ± 1,5°C, 70 % rel. Feuchte ± 10 %) aufbewahrt. Die Wirkungskontrolle erfolgt nach 24 Stunden und 48 Stunden (larvizide Wirkung). Nach dem Auswandern der Larven (ca. 72 h) werden die Glasröhrchen entfernt und gelochte Kunststoffdeckel auf die Becher gesetzt. Nach 1½-facher Entwicklungsdauer (Schlupf der Kontrollfliegen) werden die geschlüpften Fliegen und die Puppen/Puppenhüllen ausgezählt.

Als Kriterium für die Wirkung gilt der Eintritt des Todes bei den behandelten Larven nach 48 h (larvizider Effekt), bzw. die Hemmung des Adultschlupfes aus den Puppen bzw. die Hemmung der Puppenbildung. Als Kriterium für die in-vitro-Wirkung einer Substanz gilt die Hemmung der Flohentwicklung bzw. ein Entwicklungsstillstand vor dem Adulten-Stadium. Dabei bedeutet 100 % larvizide Wirkung, daß nach 48 Stunden alle Larven abgestorben sind. 100 % entwicklungsinhibitorische Wirkung bedeutet, daß keine adulten Fliegen geschlüpft sind.

In diesem Test hatten z.B. die Verbindungen gemäß den Herstellungsbeispielen 3, 5 und 7 bei einer beispielhaften Wirkstoffkonzentration von 1 000 ppm eine Wirkung von 100 %.

### Beispiel H

### Test mit Boophilus microplus resistent / SP resistenter Parkhurst-Stamm

| | |
|---|---|
| Testtiere | Adulte gesogene Weibchen |
| Lösungsmittel | Dimethylsulfoxid |

20 mg Wirkstoff werden in 1 ml Dimethylsulfoxid gelöst, geringere Konzentrationen werden durch Verdünnen mit dem gleichen Lösungsmittel hergestellt.

Der Test wird in 5-fach Bestimmung durchgeführt. 1 µl der Lösungen wird in das Abdomen injiziert, die Tiere in Schalen überführt und in einem klimatisierten Raum aufbewahrt. Die Wirkung wird über die Hemmung der Eiablage bestimmt. Dabei bedeutet 100 %, daß keine Zecke gelegt hat.

In diesem Test hatte z.B. die Verbindung gemäß Herstellungsbeispiel 1 bei einer beispielhaften Wirkstoffkonzentration von 20 µg/Tier eine Wirkung von 100 %.

## Patentansprüche

1. Verbindungen der Formel (I)
CF₂=CX-CH₂-Het (I),
in welcher
X für Wasserstoff oder Halogen steht,
Het für einen der Reste 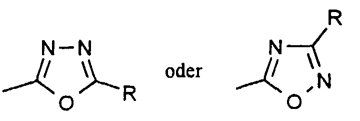 steht und
R für jeweils gegebenenfalls substituiertes Alkyl, Aryl, Aralkyl oder Hetaryl steht.

2. Verfahren zur Herstellung von Verbindungen der Formel (I) gemäß Anspruch 1, dadurch gekennzeichnet, daß man
A) Amidoxime der Formel (II) 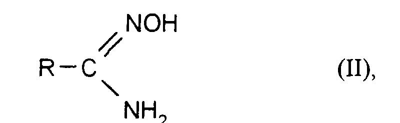 in welcher
R die in Anspruch 1 angegebene Bedeutung hat,
mit Säurechloriden der Formel (III)
CF₂=CX-CH₂-COCl (III),
in welcher
X die Anspruch 1 angegebene Bedeutung hat,
gegebenenfalls in Gegenwart eines Verdünnungsmittels und gegebenenfalls in Gegenwart einer Base umsetzt und die so erhaltenen Zwischenprodukte der Formel (IV) 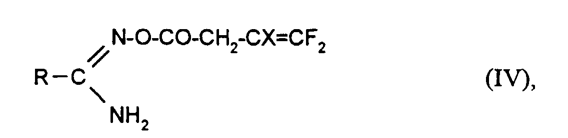 in welcher
R und X die oben angegebene Bedeutung haben,
gegebenenfalls isoliert und gegebenenfalls in Gegenwart einer Base cyclisiert,
oder
B) Carbonsäurehydrazide der Formel (V)
R-CONH-NH₂ (V),
in welcher
R die oben angegebene Bedeutung hat,
mit Säurechloriden der Formel (III)
CF₂=CX-CH₂-COCl (III),
in welcher
X für Wasserstoff oder Halogen steht,
in Gegenwart eines Verdünnungsmittels und in Gegenwart einer Base zu Verbindungen der Formel (VI)
R-CONH-NH-CO-CH₂-CX=CF₂ (VI)
in welcher
R und X die oben angegebene Bedeutung haben,
umsetzt, diese gegebenenfalls isoliert und gegebenenfalls in Gegenwart eines Verdünnungsmittels und gegebenenfalls in Gegenwart eines wasserabspaltenden Mittels cyclisiert.

3. Schädlingsbekämpfungsmittel, gekennzeichnet durch einen Gehalt an mindestens einer Verbindung der Formel (I) gemäß Anspruch 1.

4. Verwendung von Verbindungen der Formel (I) gemäß Anspruch 1 zur Bekämpfung von Schädlingen.

5. Verfahren zur Bekämpfung von Schädlingen, dadurch gekennzeichnet, daß man Verbindungen der Formel (I) gemäß Anspruch 1 auf Schädlinge und/oder ihren Lebensraum einwirken läßt.

6. Verfahren zur Herstellung von Schädlingsbekämpfungsmitteln, dadurch gekennzeichnet, daß man Verbindungen der Formel (I) gemäß Anspruch 1 mit Streckmitteln und/oder oberflächenaktiven Mitteln vermischt.

7. Verwendung von Verbindungen der Formel (I) gemäß Anspruch 1 zur Herstellung von Schädlingsbekämpfungsmitteln.

## Claims

1. Compounds of the formula (I)
CF₂=CX-CH₂-Het (I),
in which
X represents hydrogen or halogen,
Het represents one of the radicals 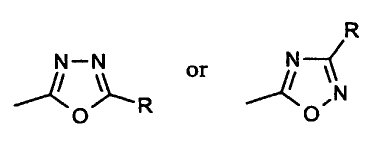 and
R represents respectively optionally substituted alkyl, aryl, aralkyl or hetaryl.

2. Process for preparing compounds of the formula (I) according to Claim 1, characterized in that
A) amidoximes of the formula (II) 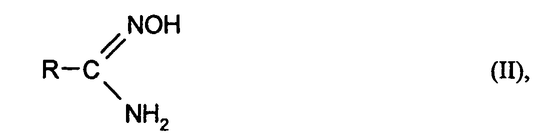 in which
R is as defined in Claim I
are reacted with acyl chlorides of the formula (III)
CF₂=CX-CH₂-COCl (III),
in which
X is as defined in Claim 1,
if appropriate in the presence of a diluent and if appropriate in the presence of a base, and the resulting intermediates of the formula (IV) 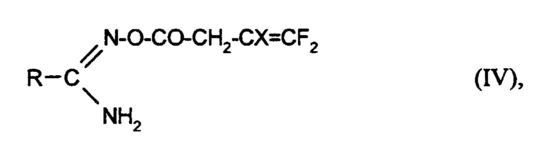 in which
R and X are each as defined above
are, if appropriate, isolated and, if appropriate, cyclized in the presence of a base,
or
B) carbohydrazides of the formula (V)
R-CONH-NH₂ (V),
in which
R is as defined above
are reacted with acyl chlorides of the formula (III)
CF₂=CX-CH₂-COCl (III),
in which
X represents hydrogen or halogen,
in the presence of a diluent and in the presence of a base to give compounds of the formula (VI)
R-CONH-NH-CO-CO₂-CX=CF₂ (VI)
in which
R and X are each as defined above,
and these compounds are, if appropriate, isolated and, if appropriate, cyclized in the presence of a diluent and, if appropriate, in the presence of a dehydrating agent.

3. Pesticides, characterized in that they comprise at least one compound of the formula (I) according to Claim 1.

4. Use of compounds of the formula (I) according to Claim 1 for controlling pests.

5. Method for controlling pests, characterized in that compounds of the formula (I) according to Claim 1 are allowed to act on pests and/or their habitat.

6. Process for preparing pesticides, characterized in that compounds of the formula (I) according to Claim 1 are mixed with extenders and/or surfactants.

7. Use of compounds of the formula (I) according to Claim 1 for preparing pesticides.

## Revendications

1. Composés de formule (I)
CF₂+CX-CH₂-Het (I),
dans laquelle
X représente l'hydrogène ou un halogène,
Het est l'un des restes 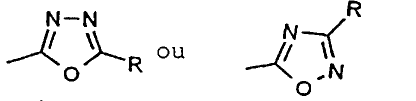
R représente un groupe alkyle, aryle, aralkyle ou hétaryle, chacun étant éventuellement substitué.

2. Procédé de production de composés de formule (I) suivant la revendication 1, caractérisé en ce que
A) on fait réagir des amidoximes de formule (II) 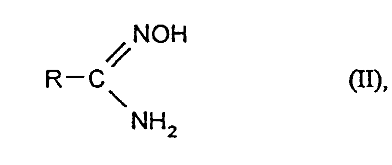 dans laquelle
R a la définition indiquée dans la revendication 1,
avec des chlorures d'acides de formule (III)
CF₂+CS-CH₂-COCl (III),
dans laquelle
X a la définition indiquée dans la revendication 1, le cas échéant en présence d'un diluant et en la présence éventuelle d'une base et on isole éventuellement les produits intermédiaires ainsi obtenus de formule (IV) 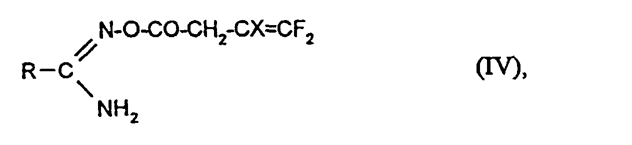 dans laquelle
R et X ont la définition indiquée ci-dessus et on les cyclise éventuellement en présence d'une base,
ou bien
B) on fait réagir des hydrazides d'acides carboxyliques de formule (V)
R-CONH-NH₂ (V),
dans laquelle
R a la définition indiquée ci-dessus,
avec des chlorures d'acides de formule (III)
CF₂=CX-CH₂-COCl (III),
dans laquelle
X représente l'hydrogène ou un halogène,
en présence d'un diluant et en la présence d'une base pour obtenir des composés de formule (VI)
R-CONH-NH-CO-CH₂-CX=CF₂ (VI),
dans laquelle
R et X ont la définition indiquée ci-dessus,
on isole éventuellement ces composés et on les cyclise le cas échéant en présence d'un diluant et, éventuellement, en présence d'un agent déshydratant.

3. Compositions pesticides, caractérisées par une teneur en au moins un composé de formule (I) suivant la revendication 1.

4. Utilisation de composés de formule (I) suivant la revendication 1 pour combattre des parasites.

5. Procédé pour combattre des parasites, caractérisé en ce qu'on fait agir des composés de formule (I) suivant la revendication 1 sur les parasites et/ou sur leurs milieux.

6. Procédé de préparation de compositions pesticides, caractérisé en ce qu'on mélange des composés de formule (I) suivant la revendication 1 avec des diluants et/ou des agents tensio-actifs.

7. Utilisation de composés de formule (I) suivant la revendication 1 pour la préparation de compositions pesticides.
